# EUROPEAN PATENT SPECIFICATION

(11) **EP 4 327 331 B1**
(45) Date of publication and mention of the grant of the patent: **30.10.2024**
(21) Application number: 22721788.2
(22) Date of filing: 12.04.2022
(51) Int. Cl.: G16H 20/70, A61B 5/16, G16H 40/20, G16H 30/20, G16H 40/63, G16H 40/67

(54) **ENTERTAINMENT CONTENT PROVIDER**
UNTERHALTUNGSINHALTSANBIETER
FOURNISSEUR DE CONTENU DE DIVERTISSEMENT

(30) Priority: 20.04.2021 NL 2028017
(43) Date of publication of application: 28.02.2024
(73) Proprietor: Koninklijke Philips N.V., 5656 AG Eindhoven (NL)
(72) Inventor: HEUVELINK-MARCK, Annerieke, 5656 AG Eindhoven (NL); HUIJBERS, Willem, 5656 AG Eindhoven (NL); HEIJMAN, Edwin, 5656 AG Eindhoven (NL); NAUTS, Sanne, 5656 AG Eindhoven (NL)
(74) Representative: Philips Intellectual Property & Standards
(86) International application number: PCT/EP2022/059668
(87) International publication number: WO 2022/223355

(56) References cited:
- EP-A1- 3 575 812
- US-A1- 2020 233 485
- US-B1- 10 783 799

## Description

### FIELD OF THE INVENTION

The present invention generally relates to an entertainment content provider and corresponding method and computer program product for providing an entertainment sequence to a patient during a medical procedure.

### BACKGROUND OF THE INVENTION

To reduce a patient's anxiety and improve their experience during a medical procedure such as Magnetic Resonance Imaging (MRI) or other (tomographic and non-tomographic) diagnostic imaging scans (e.g. X-ray imaging, computed tomography, positron emission tomography, single-photon emission computed tomography, ultrasound imaging, etc.), image-guided treatment or therapies, radiotherapy procedures, proton therapy or some interventional procedures, entertainment may be provided during the medical procedure, such as audio (music, audiobooks), calming images or a movie, for example, on an entertainment screen or through goggles. Currently this entertainment content is not adapted to facilitate, or optimize, the medical procedure that the patient is undergoing.

In known entertainment providing systems entertainment content provided to patients is intended to improve the overall patient experience. Uncurated content is often problematic, e.g. online videos or commercial movies, as it tends to induce motion via arousing content, laughter, distress or sudden visual onset that may cause body or eye movements. These can be detrimental to the medical procedure, e.g. by worsening medical image quality or it may negatively affect a treatment, especially when they occur at sensitive times during the procedure, for example in image-guided therapy.

Curated content, like for instance movies for patients during a medical procedure, overcome this issue partially, as they are designed to be overall relaxing, not causing any arousal or undesired motion. Yet, this still has several disadvantages. For instance, this type of content may not be optimally engaging (e.g., it can be perceived as boring or uninteresting). Also, it may be difficult for patients to remain perfectly still during an entire exam or treatment. As such, providing them with opportunities to take a break/move at times, may reduce overall motion during the procedure, especially during long procedures. Furthermore, content that facilitates a continuously relaxed state during the examination may not be optimal in all parts of the examination. After all, not all procedures or procedural steps (e.g. individual MRI sequences) are the same. Curated content does not facilitate, motivate or prevent any behavior that is (un)desired for the outcome measures of the procedure at any given time.

US2020/233485A1 discloses a VR system for use during a medical procedure that offers entertainment to a patient.

EP3575812A1 discloses matching entertainment sounds provided to a patient to pulse sequences of an MRI procedure.

Therefore, there is opportunity to improve patient experience, image quality, treatment effectivity and the medical workflow by real-time adapting the content to what occurs during the medical procedure.

### SUMMARY OF THE INVENTION

Embodiments according to the present invention are directed to an entertainment content provider for providing an entertainment sequence to a patient during a medical procedure, wherein the medical procedure comprises a succession of procedural steps. The entertainment content provider comprises a procedural step database comprises a catalogue of procedural steps associated with the medical procedure and wherein each procedural step in the catalogue includes procedural step metadata including a procedural step identifier; a procedural step length; and at least one risk factor that is based on a previously determined detrimental effect of patient behavior on the outcome of the medical procedure. The entertainment content provider further comprises a curated content database, comprising a catalogue of entertainment blocks, wherein each entertainment block includes: entertainment content; and metadata including: an entertainment block length; and predetermined expected effect data on one or more procedural steps from the procedural step database. The entertainment content provider further comprises a content scheduler, configured to receive medical procedure data of the medical procedure, comprising data relating to the procedural steps in the succession of procedural steps, including an order of the procedural steps in the succession of procedural steps; consult the procedural step database and select the procedural step options corresponding with the successive procedural steps of the medical procedure; consult the curated content database and select entertainment blocks by matching the risk factor of the selected procedural options with a most suitable predetermined expected effect data of an entertainment block; create an entertainment content schedule by listing the selected entertainment blocks based on the order of the procedural steps in the succession of procedural steps; and the entertainment content provider comprises a content executor, configured to: receive the entertainment content schedule from the content scheduler; retrieve the selected entertainment blocks in the content schedule from the curated content database; provide entertainment content to the patient during the medical procedure by presenting the retrieved entertainment blocks according to the entertainment content schedule.

As such an entertainment sequence may be created that fits the medical procedure in length and will provide a satisfying complete entertainment experience to the patient.

In an embodiment the content scheduler is further configured to also select entertainment blocks by matching or adapting an entertainment block length to a procedural step length. As such there is more freedom to choose between entertainment blocks as they don't need to exactly fit the procedural step length.

In an embodiment the entertainment blocks further comprise metadata relating to storyline priority and wherein the content scheduler is further configured to also select entertainment blocks based on the storyline priority metadata.

As such the storyline will have a proper flow and order and will enhance the satisfactory nature of the entertainment sequence.

In an embodiment the entertainment block length is fixed, adaptable or dynamic.

As such there may be multiple options available to create an entertainment sequence that is optimally matched to the medical procedure.

In an embodiment the entertainment block further includes patient target group information indicating for which patient target group the entertainment content is most suitable, for instance a patient target group based on age or age group, gender, cognitive ability, eye sight, hearing, literacy, diagnosis, special interests and the like and the content scheduler is further configured to also select entertainment blocks based on the patient target group information.

As such the entertainment content may be selected that is most suitable and applicable for each patient that is undergoing the medical procedure.

In an embodiment the content scheduler is further configured to: receive updated medical procedure data before or during the medical procedure, comprising updated data relating to the procedural steps in the succession of procedural steps; compare the updated medical procedure data with the medical procedure data; and in case any of the procedural steps has changed, has been removed and/or added: consult the procedural step database and select the procedural step options relating to the successive procedural steps of the updated medical procedure; consult the curated content database and select entertainment blocks based on the selected procedural options; update the entertainment content schedule by listing the selected entertainment blocks based on the order of the procedural steps in the succession of procedural steps of the updated medical procedure.

As such the entertainment sequence may be updated, in real-time, to adjust to the updated medical procedure. This ensures that the entire entertainment sequence remains satisfying to the patient and is not ended prematurely or is not finished or is not matching the procedural steps anymore. In an embodiment the entertainment content provider further comprises a patient behavior module, comprising: a patient behavior data receiver configured to receive patient behavior data relating to the medical procedure; a patient behavior data provider for providing the patient behavior data to the content scheduler; and wherein the content scheduler is further configured to receive the patient behavior data from the patient behavior module and to consult the curated content database and select entertainment blocks also based on the patient behavior data.

As such a more individual or more suitable entertainment sequence may be chosen, as it may be better tuned to the patient behavior characteristics.

In an embodiment the patient behavior data is previously obtained patient behavior data, library patient data, modeled patient behavior and/or real-time acquired patient behavior data.

As such the patient behavior data may be obtained from various sources that are available.

In an embodiment a neural network is configured to analyze the previously obtained patient behavior data, library patient data, modeled patient behavior data and/or real-time acquired patient behavior data to generate expected patient behavior data forming or replacing the patient behavior data.

As such the neural network will learn with each new entertainment selection session and will therefore improve over time to create an increasingly better entertainment sequence with each additional patient.

In an embodiment the content executor is further configured to receive updated medical procedure data; adapt the entertainment schedule by adapting, removing or adding at least one entertainment content blocks; and provide the adapted entertainment content.

As such it is ensured that the patient receives the most recent and correct entertainment blocks.

In an embodiment the medical procedure is a magnetic resonance imaging (MRI) procedure and the succession of procedural steps includes at least one MRI sequence.

The invention is particularly useful for MRI procedures as they are often perceived to be intimidating and/or claustrophobic. Therefore providing an engaging entertainment sequence helps the patient to be more relaxed, resulting in better results.

The present invention is further directed towards a corresponding method and computer program product.

Still further aspects and embodiments of the present invention will be appreciated by those of ordinary skill in the art upon reading and understanding the following detailed description. Numerous additional advantages and benefits will become apparent to those of ordinary skill in the art upon reading the following detailed description of preferred embodiments.

### BRIEF DESCRIPTION OF THE DRAWINGS

The present invention is illustrated by drawings of which
Fig. 1 shows a schematic depiction of an MRI system with an entertainment system and an entertainment content provider according to the presently claimed invention.
Fig. 2 shows a schematic depiction of an entertainment content provider according to an embodiment of the presently claimed invention and associated further equipment and personnel.
Fig. 3 shows an exemplary timeline of API instructions a from the MRI scanner to the entertainment content provider coupled to the MRI system behavior to illustrate the presently claimed invention.
Fig. 4 shows an exemplary workflow of a method according to an embodiment of the presently claimed invention.

The invention may take form in various components and arrangements of components, and in various process operations and arrangements of process operations. The drawings are only for the purpose of illustrating preferred embodiments and are not to be construed as limiting the invention. To better visualize certain features may be omitted or dimensions may be not be according to scale.

### DETAILED DESCRIPTION OF EMBODIMENTS

The presently claimed invention improves medical procedures, such as MRI imaging, by adapting the entertainment content to the specific risks and timing of the medical procedure steps (e.g. sequences in a scan or examination) to increase the chance that a patient behaves in such a way that the procedure is successful (or in other words, decrease risk of detrimental patient behavior). While doing so, the claimed invention takes certain characteristics of human behavior in the bore into account, e.g. that there is a maximum time limit people can hold their breath or hold still otherwise, that over time attention decreases while the risk for motion increases, that certain people prefer certain content over others etc.

Particular examples one can think of is that when motion causes a high risk for a successful MRI sequence, radiotherapy step or interventional procedure step, very engaging scenes (that capture a patient's attention and reduce motion) are shown. Alternatively, challenging cognitive tasks or puzzles can be presented during these motion-high-risk moments as it is known that offering challenging cognitive tasks leads to reduced motion. In contrast, indication of performance or answers to the puzzle that may lead to a physical response can be presented after the procedure, when there is no risk associated with motion. Similarly, funny content designed to reduce anxiety and increase engagement, but that may induce laughter, should only be scheduled at intervals between procedural steps. Another example is when high or slow cardiac activity is a risk to a particular cardiac scan or interventional procedure, content is selected that is either very likely or not likely at all to cause an adrenaline rush. For instance, in specific functional MRI (fMRI) sequences, eye movements are a risk to good outcomes, content can be selected that clearly fixes eye gaze / focus on one point, leading to minimal eye motion and thereby enlarging the chance of a successful scan.

The presently claimed and described invention is directed towards an adaptive entertainment content providing system and method to stimulate behavior of the patient during a medical scan, examination or treatment that increases the chance of a successful medical procedure with as little as patient discomfort as possible.

The presently claimed invention focuses on scheduling content in isolation, leading to optimal behavior for each part of the scan. From a patient experience perspective, it is desired to schedule content that embeds an engaging, immersive narrative storyline instead of non-curated content with unpredictable effects on a patient or with generic curated content without a storyline. Also, it would be undesirable to provide entertainment content with a storyline that won't complete during the procedure or that would complete prematurely, as this may have adverse effects on a patient's mood and compliance to obtain an optimal scan or therapy result.

In the following the claimed entertainment content providers illustrated using an MRI medical scan, but the invention is certainly not limited to this or other types of medical scans, diagnostic or therapeutic procedures. It is suitable for any medical procedure in which motion and/or patient comfort may influence the outcome of the procedure, such as other medical diagnostic imaging such as computed tomography, x-ray, ultrasound, positron emission tomography, single photon emission spectroscopy. Further the claimed invention is suitable for other medical procedures that require a patient to be not, locally or mildly sedated, such as (minimally) invasive therapeutic or diagnostic medical procedures, such as catheterizations or (minimally) invasive surgery or for radiation treatment, such as radiation therapy using focused radiation beams.

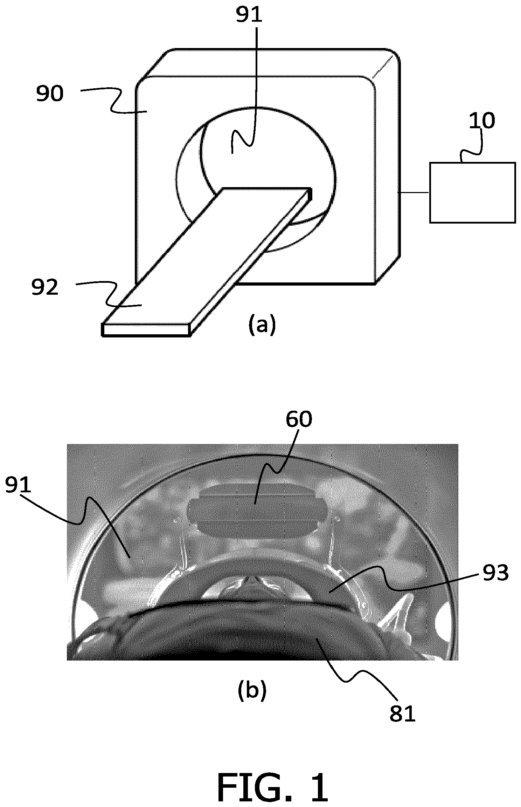

Fig. 1 depicts an example of an in-bore entertainment device for patients undergoing an MRI scan. In an MRI device 90 a patient on a patient support 92 is placed in the bore 91 of the MRI device 90, which uses strong magnetic fields, magnetic field gradients and radio waves (picked up by receiving coils 93) and a processing unit (not shown) to generate images of the organs in the patient's body. A schematic set-up is shown in Fig. 1a and photograph of a patient inside a bore 91 is shown in Fig. 1b.

As is immediately clear from Fig. 1b, the bore 91 is relatively small and the head coil 93 is placed closely over the patient's head, creating a very narrow space that may induce claustrophobic feelings in the patient. Furthermore, the MRI scan may take between a few minutes to even an hour and a half or more in which the patient has to lie still, while the procedure involves regular and irregular loud noises close to the patient's ears. All these factors contribute to the patient feeling uncomfortable, claustrophobic, impatient or even scared. This is particularly relevant for pediatric, psychiatric and dementia patients.

To provide distraction and relief an in-bore entertainment system 10 that provides entertainment to the patient during the scan may be used. The entertainment system may for instance be an in-bore display or a mirror or screen onto which the entertainment content is projected. The entertainment system 10 usually includes headphones (not shown), which also reduce ambient noises and may be used to communicate with the patient 20. The entertainment system 10 may also include means for providing visual information and entertainment, such as an (MRI-compatible) in-bore screen, a screen placed outside of the bore that is seen through a mirror (as shown in Fig. 1b), a screen on the ceiling of the imaging room, 2D or 3D (MRI-compatible) (virtual reality) goggles, a projected screen and the like, which again also may be used to provide the patient with instructions and/or information relating to the scan (e.g. time left, a scan progress bar) as well as entertainment.

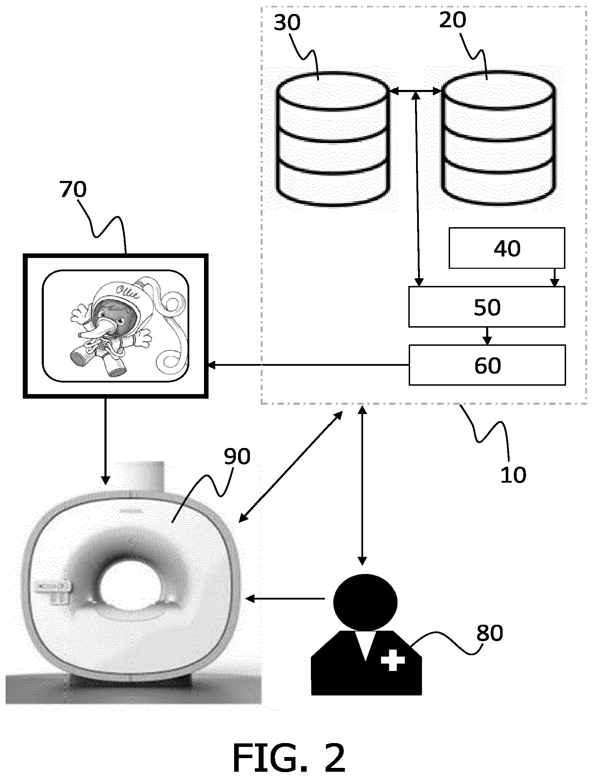

Fig. 2 shows simplified schematic overview of components of the presently claimed entertainment content provider 10.

A scan plan, or Exam Card, is prepared and/or received by an MRI operator 80, e.g. a qualified radiologist, technologist, physician or nurse. The scan plan comprises various procedural steps, i.e. MRI sequences, that are selected to provide an optimal scan result imaging (a part of) a patient.

The scan plan is input in a control unit of the MRI device 90 and, in the presently claimed invention, is also input in the entertainment content provider10. Further input to the entertainment content provider 10 may be patient specific information, such as a patient target group based on age or age group, gender, cognitive ability, special interests of the patient and the like.

The entertainment content provider 10 comprises a procedural step database 20 that comprises a catalogue of, preferably all, procedural steps (MRI sequences) that the MRI device 90 may perform. Each procedural step (MRI sequence) in the procedural step database comprises metadata relating to a procedural step identifier that identifies the procedural step (for instance a sequence name or other identifier). The metadata further includes a length of the procedural step (sequence), preferably as precise as possible. In some cases, the procedural step length may be adaptable and the MRI operator 80 may provide such suitable additional input to the entertainment content provider 10. Particularly for static MRI sequences the length is a suitable metric to include in the metadata. However, for adaptive (e.g. cardiac, respiratory) MRI sequences the length parameter stored in the metadata is an indicative length, for instance based on an average, minimum or maximum length that was previously determined for said procedure. There may be several relevant variables and options for each procedural step dependent on the particular patient undergoing the medical procedure or on particular settings or equipment used in the procedure.

Further the metadata includes at least one risk factor. This risk factor is based on a previously determined detrimental effect of patient behavior on the outcome of the procedural step. For instance, the risk factor may be based on sensitivity to slow and/or fast patient motion. A procedural step may have more than one risk factor associated with it and the value of the risk factor may vary over the step length (e.g. for MRI sequences this could be related to the k-lines being acquired; k-line 0 is more sensitive to motion than k-line 100).

For each type of procedural step (MRI sequence), the degree to which certain behavior is detrimental to the image quality is determined and annotated (manually by experts or automatically deduced from scan specifics, for instance using A.I. technology). These annotations are then translated into risk factors that may, for instance, be expressed in different granularity or types (e.g. low, mid, high vs. 10%, 30%, 50% vs. 0.01 mm, 0.1 mm). The annotation may hold for a full procedural step (sequence) or be further specified to parts of the procedural step (sequence), e.g. patient motion delivers a high risk during a specific timeslot, but only low risk during the rest of the sequence. Each procedural step may have the most suitable risk factor and they do not require to be the same type or range for all procedural steps.

The entertainment content provider 10 further comprises a curated content database 30 that comprises a catalogue of entertainment blocks. An entertainment block contains part of an entertainment storyline, for instance a movie scene or part thereof, and a sequence of entertainment blocks form a full entertainment sequence with, preferably, a storyline that is optimized to the procedural steps of the medical procedure and is engaging to the patient undergoing the MRI exam. Entertainment blocks may comprise content that only is relevant to a storyline, but may also comprise functional information, such as breathing guidance or content that is intended to calm a patient or evoke an action, or warn that a part of the procedure (e.g. a scan or sequence) starts, a reminder to stay still, etc., preferably embedded as part of the entertainment storyline, for instance a character may speak directly to the viewer and provide information or instructions or it may be induced by visuals and/or audio, for instance a calming movie and/or quiet music or an object that the viewer follows with his eyes. An engaging entertainment sequence may also consist of shorter individual sequences that transition into each other directly or through intermediate blocks or sequences (for instance to provide the patient with instructions for the next procedural step) or give the patient time to recover from the previous step).

Each entertainment block further contains metadata that includes length of the entertainment block (for instance in seconds or minutes) and if this length is adaptable or not and if so, preferably the metadata comprises a minimum and maximum length. Each entertainment block further includes predetermined expected effect data on one or more procedural steps form the procedural step database 20. The predetermined expected effect data comprises data specifying what effect a specific entertainment block is expected to have on the patient (i.e. calming the patient down, regulate breathing, provoke an action, etc.).

The curated content may either be of fixed, adaptable or dynamic length (within practical and content-determined boundaries). With fixed length it is meant that the entertainment block has a non-adaptable length, for instance because it would have a large (negative) impact on the flow of an overall storyline or may fully contain essential information for the storyline. With adaptable or dynamic length it is meant that an entertainment block may be shortened or extended, for instance by excising or attaching entertainment content from or to the entertainment block. For instance, an entertainment block may have two or more sub-sections that contain parts of a full scene. Each or some of these sub-sections may be marked as essential to the storyline and others may be considered optional. Of course the sub-sections may alternatively be separate blocks altogether. In this way the entertainment block may be matched to a desired length to correspond as closely with a procedural step length that is performed simultaneously with said entertainment block. Alternatively, or additionally, the length of an entertainment block may be played (slightly) faster or slower to match the desired length of the entertainment block. In that case care must be taken to make sure the entertainment content is not overly noticeable influenced by the change in playback speed, as this may solicit an undesired reaction from the patient. A further example may be an entertainment block having an end scene (e.g. driving away in the distance) that may last 5 or 30 seconds and anytime in between to adapt to the procedure step length.

The predetermined expected effect data is obtained by curating the content manually by experts or learned by observing people's behaviors in response to the content. The content of the entertainment blocks is annotated with a degree in which it is (un)likely to cause certain behavior.

For each entertainment block the expected effect data for each procedural step in the procedural step database can be deduced. Alternatively, for blocks were that is possible, such entertainment blocks may include, in their metadata, predetermined expected effect data for each procedural step in the procedural step database. This may in some cases be general (i.e. an entertainment block is expected to calm a patient down, cause laughter, etc.) or very specifically related to a certain procedural step (i.e. focus the gaze to a certain area). The predetermined expected effect data preferably matches the risk factor types used for relevant entertainment blocks as this will make matching entertainment blocks with procedural step risk factors more efficient. This matching process is preferably performed by an algorithm, most preferably an A.I. algorithm.

Preferably the entertainment blocks further comprise metadata relating to storyline priority. To obtain an engaging storyline the entertainment blocks are to be played in a logical sequence (e.g. set-up, development, conclusion of the storyline) and the storyline metadata may indicate that the entertainment, for instance, starts or ends a storyline, should be played (directly) before or after another entertainment block, contains information essential to the storyline or is an optional entertainment block that may be used to expand the main storyline (e.g. a character moment, a side-adventure, an ambient moment, etc.) during a procedure that is longer than only the entertainment blocks that are essential to the storyline.

Preferably the entertainment blocks in the curated content database 30 are searchable by or include information relating to patient target group information that indicates for which patient target group the entertainment content is most suitable. For instance, a patient target group may be based on age or age group, gender, cognitive ability, eyesight, hearing, literacy, diagnosis, psychiatric issues, cognitive issues, special interests and the like. Preferably the MRI operator 80 is aware of the patient's relevant characteristics and preferences (for instance by discussing this with the patient and provide the patient with selectable options) and can as such make a first selection between main storyline options. This reduces the manual and/or computational effort to match entertainment blocks with procedural steps as many of the entertainment blocks not suited to the patient target group or the selected storyline may be disregarded.

The entertainment content provider comprises a content scheduler 50 that selects the most suitable entertainment blocks for a medical procedure and provides a schedule of the entertainment blocks to match the medical procedure (in this example an MRI scan) and its steps (in this example the MRI sequences).

The content scheduler 50 analyzes the medical procedure and its successive procedural steps and all other relevant input, such as content preferences for a patient target group as mentioned or an individual preference selected by the patient. Furthermore, the content scheduler 50 may optionally receive relevant information from a patient behavior module 40, which provides further relevant data that may be relevant for selecting specific entertainment blocks.

The patient behavior module 40 may use patient-specific information received from a medical specialist (e.g. the MRI operator 80) or from the patient through a (digital) input questionnaire. For instance, the patient specific information may comprise a patient risk factor indicating a likeliness for a patient to have patient behavior that is expected or not to be problematic in various levels. Alternatively, the patient behavior may also be obtained from library patient data from which (averaged) patient behavior data from similar patients is retrieved (e.g. based on similar patient target groups as mentioned previously). Also, the patient behavior data may be modeled based on patient specific, library data or a combination of both. And, preferably, real-time patient data may be used to determine (or update) the patient behavior data. For instance, cardiac information, sweat detection, optical camera observations, movement sensors and the like may be used by the patient behavior module 40 to determine and predict stress or movement with the patient. Likewise, observations by medical personnel may be used by the patient behavior module 40 to determine and predict patient behavior data. The patient behavior module 40 preferably makes use of a recommender system to efficiently determine and/or predict patient behavior data and preferably has machine learning capabilities to improve the patient behavior data determination or prediction when more data from more patients is processed over time

Based on the received input the content scheduler 50 consults the procedural step database 30 and selects the procedural steps from the catalogue that correspond with the successive procedural steps (sequences) in the medical procedure (MRI scan) that is to be performed. The content scheduler 50 is configured to read and analyze the metadata of the procedural steps in the procedural step database 20 and the entertainment blocks in the curated content database 30.

Next, the content scheduler 50 consults the curated content database 30 to select the most suitable entertainment block for each procedural step. The content scheduler 50 takes into account the risk factor of each procedural step and tries to find and select an entertainment block with predetermined expected effect data that is most suited to reduce the risk of detrimental patient behavior. For instance, in case a procedural step is particularly sensitive to motion, then an entertainment block that is expected to have a particularly engaging or calming effect may be selected. Or, if a patient is required to have his eyes fixed in a certain direction, then an entertainment block may be selected that draws the eyes to the desired direction.

Preferably the order of the procedural steps is taken into account and a preferred storyline is known, such that the content scheduler 50 efficiently can pick entertainment blocks from a limited pool of entertainment blocks. Furthermore, the content scheduler 50 preferably takes entertainment block storyline metadata into account to create an engaging storyline with a beginning and a satisfying ending. Each selected entertainment block must match the length of its corresponding procedural step. If no suitable entertainment block of the correct length can be selected, then, preferably the entertainment block length is adapted, for instance by changing the playback speed, or two or more successive entertainment blocks are selected to cover one procedural step, provided that the additional blocks obviously are not detrimental, or preferably, actually are particularly suitable to achieve the desired effect on patient behavior. Alternatively or additionally, there may be `open ended' entertainment blocks that have a variable length, for instance a scene in which a landscape or other non-storyline specific scene or a scene in which a character moves (e.g. dances, walks, flies, travels, etc.) is shown for a certain amount of time and which can be easily shortened or lengthened without affecting the storyline.

Preferably the curated content database 30 comprises multiple interchangeable entertain blocks to achieve a certain effect (within and matching with a single storyline), such that the content scheduler 50 has an option to select the most suitable entertainment block for the desired effect and matches most optimally with the procedural step length.

Next, the content scheduler 50 creates an entertainment content schedule which lists the selected entertainment blocks in the order which they will be played during the medical procedure. This order is directly based on the order of the successive procedural steps to be performed during the medical procedure. Optionally the content scheduler may provide multiple schedules if there are multiple options with the same or similar expected results and that fit with the medical procedure and patient preferences. In such cases the patient and/or medical operator 80 may indicate which schedule should be selected by the content scheduler 50.

Preferably the content scheduler 50 also indicates the length of each entertainment block on the schedule.

In an exemplary, specific and non-limiting example of a planned MRI scan procedure the following plan call of the MRI scanner, a so-called ExamCard in the form of a JSON string, is retrieved from the medical procedure database, in this case an MRI sequence database:
*{'id': 'c315544c-c55f42e9-ba29-f86dc8165f14', 'durationlnSeconds': '18', 'progress': '0', 'state': 'Ready to Run', 'name': 'Survey: 'imagingSequence': 'FFE', 'fastImagingMode': 'TFE', 'tr': '15', 'te': '5.2', 'ti': None, 'scanMode': 'M2D', 'hasBreathHoldPeriod': 'False', 'hasTableMotion': 'False', 'isDiagnosticScan': 'True'},*
*{'id': '05b92ec1-4a9e-41ce-9ac7-d53057a6460d', 'durationlnSeconds': '2', 'progress': '0', 'state': 'ReadyToRun', 'name': 'CoilSurveyScan', 'imagingSequence': 'FFE', 'fastImagingMode': 'NO', 'tr': '5.4', 'te': '1.27', 'ti': None, 'scanMode': '3D', 'hasBreathHoldPeriod': 'False', 'hasTableMotion': 'False', 'isDiagnosticScan': 'False'},*
*{'id': 'cc6ce300-af0b-4d1d-becd-42b99a44157d', 'durationlnSeconds': '151', 'progress': '0', 'state': 'ReadyToRun', 'name': 'T2W_TSE', 'imagingSequence': 'SE', fastImagingMode': 'TSE', 'tr': '4884', 'te': '110', 'ti': None, 'scanMode': 'MS', 'hasBreathHoldPeriod': 'False', 'hasTableMotion': 'False', 'isDiagnosticScan': 'True'},*
*{'id': '7e13bc8b-419c-4abd-b332-bbf2aed083f8', 'durationlnSeconds': '308', 'progress': '0', 'state': ReadyToRun ; 'name': 'FLAIR_longTR', 'imagingSequence': 'IR', fastImagingMode': 'TSE', 'tr': '11000', 'te': '140', 'ti': '2800', 'scanMode': 'MS', 'hasBreathHoldPeriod': 'False', 'hasTableMotion': 'False', 'isDiagnosticScan': 'True'},*
*{'id': 'e07e44d7-4e3c-4fdb-b15a-305df8589436', 'durationlnSeconds': '214', 'progress': '0', 'state': 'ReadyToRun', 'name': 'T1W_SE', 'imagingSequence': 'SE', 'fastImagingMode': 'NO', 'tr': '581', 'te': '15', 'ti': None, 'scanMode': 'MS', 'hasBreathHoldPeriod': 'False', 'hasTableMotion': 'False', 'isDiagnosticScan': 'True'},*
*{'id': 'ad71ffe2-abbf-4f7f-926b-b8d1b29dc7bd', 'durationlnSeconds': '35', 'progress': '0', 'state': 'ReadyToRun', 'name': 'DWI', 'imagingSequence': 'SE', 'fastImagingMode': 'EPI', 'tr': '2942', 'te': '89', 'ti': None, 'scanMode': 'MS', 'hasBreathHoldPeriod': 'False', 'hasTableMotion': 'False', 'isDiagnosticScan': 'True'},*
*{'id': '25a2100c-724e-45f4-8956-f2a3b626874f', 'durationlnSeconds': '190', 'progress': 'O', 'state': 'Ready', 'name': 's3DI_MC', 'imagingSequence': 'FFE', fastImagingMode': 'NO', 'tr': '23', 'te': '6.9', 'ti': None, 'scanMode': '3D', 'hasBreathHoldPeriod': 'False', 'hasTableMotion': 'False', 'isDiagnosticScan': 'True'}*

The content scheduler 50 consults the procedural step (sequence) database 30 to retrieve the risk factor that indicates information on the behaviors whose chances of occurrences should be minimized during particular moments of the scan/examination.

Next, the content scheduler 50 selects entertainment blocks from the curated content database 20 that match these requirements by matching entertainment blocks that have expected effect data that reduces the risk as indicated in the risk factors. In case of multiple options it may select the entertainment block that is expected to reduce the risk the most (e.g. if the risk factor or expected effect data has several ranges of risk or effect).

The scheduler also takes into account that the duration of the content matches that of the sequence as closely as possible. This means for example that content scheduled for dynamic scans whose duration is influenced by e.g. heart rate or breathing rate, is long enough and can be cut short at any moment without causing an unexpected or unpleasant experience.

Moreover, the content scheduler 50 embeds knowledge about MRI examinations, e.g. it knows that there are moments between the sequences that can also be used to display content to the patient. In the context of this invention such pauses between medical steps (also for non-MRI procedures) are considered to be a medical procedure step as well.

In some embodiments the content scheduler 50 uses a patient behavior module 40 that uses received information of a patient to provide additional input to the content scheduler 50 and/or models expected in-bore behavior of the patient to further optimize the scheduled content by consulting patient-specific data, library data of similar subjects as the patient, previously modeled patient behavior or real-time patient behavior measured or observed during the procedure.

The patient behavior module 40 may use deep learning neural networks or other artificial intelligence algorithms to model or predict the patient's behavior.

A particular, non-limiting example of modeled patient behavior comprises two elements: First: Information on the chance of certain behavior occurring simply from the perspective of the human body (manually crafted and curated by experts or learned by observing people's behaviors). For example, the chance that a person will breathe in increases the longer they have to hold their breath. Similarly, the chance that a person blinks is increases as the time increases since the last blink. Another example is that the longer a person lies in the bore, the higher the chance of motion is. Second: Information on how a patient can be best engaged from the perspective of the human psyche. For example, repetition of the same content is likely to be less engaging, content that forms a storyline is more engaging. As an example, content that includes princesses, unicorns, mermaids, (elephant) astronauts or firemen is more likely to engage many children of age 3 to 7 then it would engage older patients.

A content executor 60 receives the entertainment content schedule from the content scheduler 50. Next the content executor accesses the curated content databases and retrieves, for instance by downloading, all entertainment blocks listed on the entertainment content schedule.

When the medical procedure starts, the content executor 60, triggered automatically or manually, plays the retrieved entertainment blocks in the order as listed on the entertainment content schedule. The content executor 60 may supply the entertainment content by having video content played on display device 70, such as a screen (directly or via a mirror) or goggles and audio content through speakers or a headphone.

Preferably, in case a retrieved entertainment block does not match a length as indicated on the schedule, the content executor 60 adapts the entertainment block such that it is extended or shortened, for instance by adapting the playback speed or adding or removing sub-scenes.

Additionally, to improve synchronization with each procedural step, given the scheduled content and the current examination status, the content executor 60 may show the scheduled content that matches with risks of a current moment in the medical procedure on the screen. For this it can use specific "prepare", "start" and "stop" calls that are send by the MRI scanner for each individual sequence and may be used to synchronize the shown content with the MRI sequences and/or pauses, in addition to the "plan" call that provides information about the status of the complete exam card.

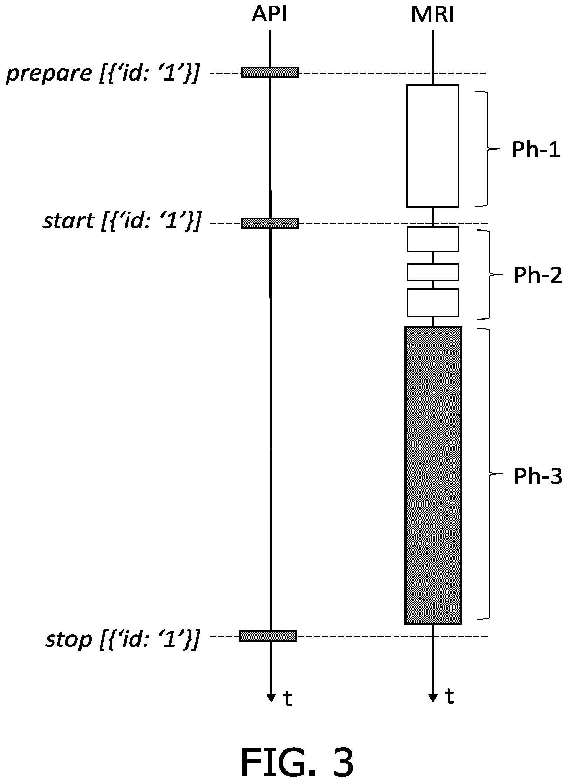

This is schematically illustrated in Fig. 3, which shows a timeline (t) diagram of for an exemplary scan sequence named 'id'. The API (Application Programming Interface) command string is shown on the left and the MRI scanner events following the successive API strings is shown on the right. Ph-1 indicates a phase that has an (auto-voice) announcement, Ph-2 indicates steps in a preparation phase and Ph-3 is the acquisition phase in which the image data is acquired. Note: The darker blocks occur always, while the white blocks are optional depending on scanner settings and status.

The procedural step database 20, the curated content database 30 and the patient behavior module 40 may be part of the entertainment content provider 10, as shown in Fig. 1, but one or more elements may also be separate modules running on the same or different processors or storage mediums.

In the following an exemplary embodiment of an MRI examination of a patient's brain is described.

Delivery of entertainment content adapted to an examination to optimize patient behavior during medical imaging is a two phase process. First, proper content should be scheduled, and secondly, it should be delivered at the right moment.

In phase 1 content is scheduled based on a scan or examination plan.

When an MRI examination is planned at the MRI scanner, the content scheduler 50 receives planned sequences (sequence names are known in the field of MR imaging), see Table 1.

**Table 1: Example of MRI examination ExamCard with several sequences including durations.**

| **ID** | **MRI Sequence** | **Duration (min)** |
|---|---|---|
| **1** | Localizer | 2 |
| **2** | T1 | 7 |
| **3** | Fieldmap fMRI | 0.5 |
| **4** | resting-state fMRI | 10 |
| **5** | Fieldmap diffusion | 0.5 |
| **6** | Diffusion-weighted MRI | 10 |
| **7** | T2 | 5 |
| **8** | Task-based fMRI | 10-30 |

Based on such ExamCard data the content scheduler 50 determines the specific procedural steps content should be scheduled for, see Table 2.

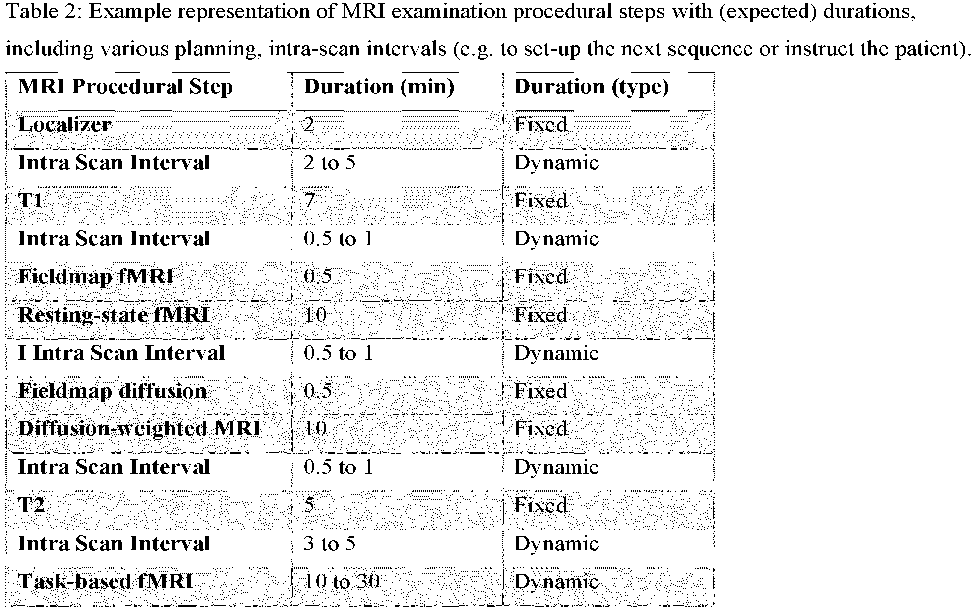

Next, the content scheduler 50 consults the procedural step dataset 20 and from the metadata of each of the procedural steps retrieves the risk factors of certain behavior for each of these procedural steps, see Table 3.

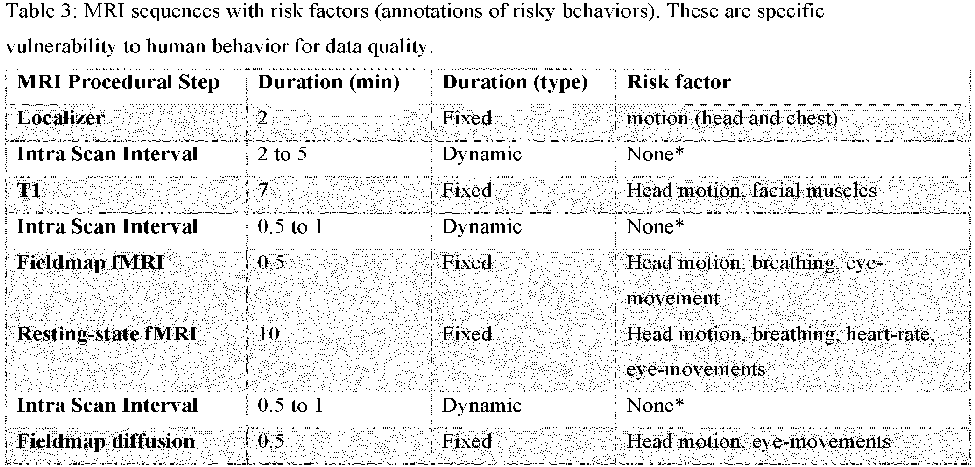

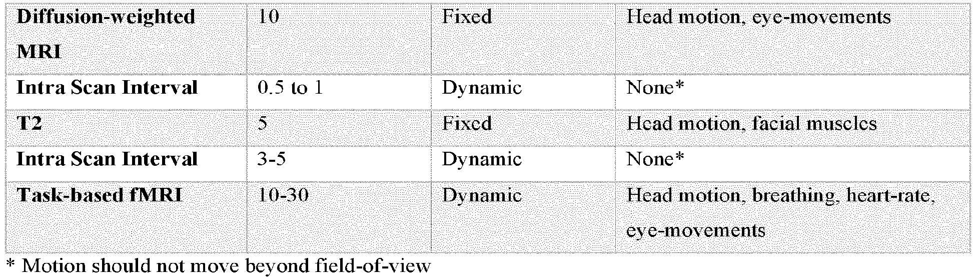

Next, the content scheduler 50 schedules content for each of defined procedural steps by consulting the curated content database 30 and match the best entertainment block for each procedural step using the metadata relating to predetermined expected effect data and comparing it to the risk factor of the entertainment block. In a particularly sophisticated embodiment the content scheduler 50 finds a balance between at the one hand minimizing the chance of behavior that is risky for the entertainment blocks, while at the other hand optimizing patient engagement. Table 4 shows the example shown in table3, but now procedural steps are matched optimal entertainment blocks. For the latter the content scheduler using input from a patient behavior model provided by a patient behavior module 40.

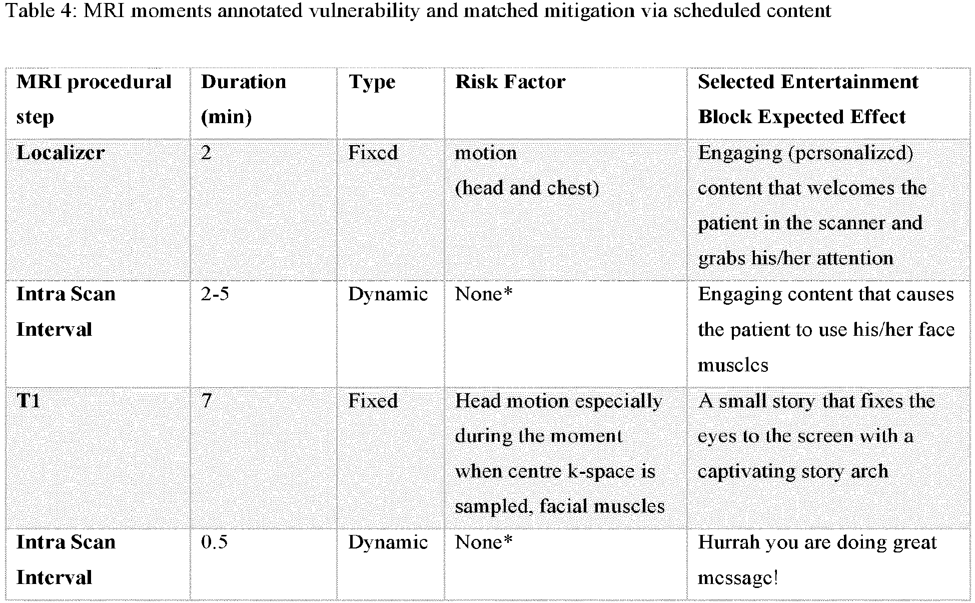

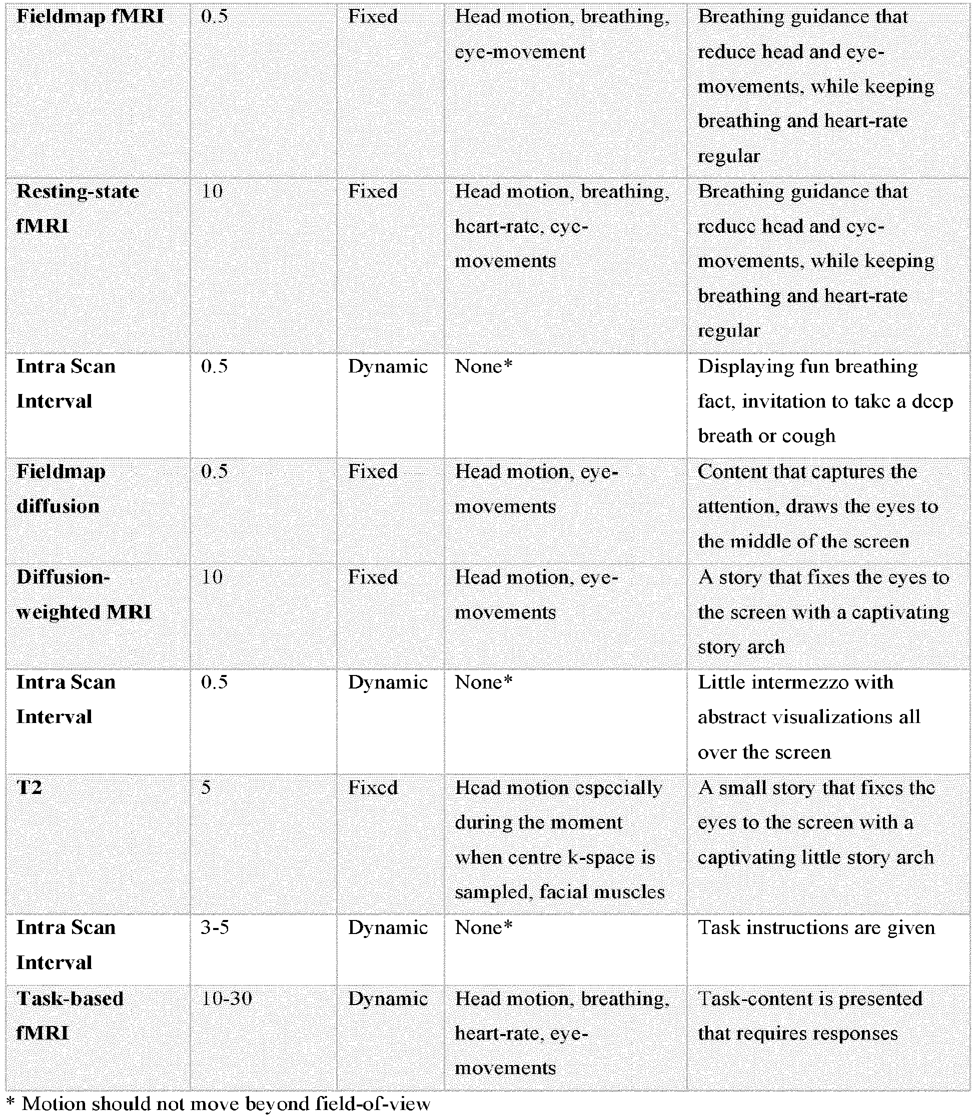

One technical approach of implementing the scheduling step resulting in Table 4 based on the knowledge presented in Table 3 is to use a constraint satisfaction problem (CSP).

A CSP is defined by:
1) a set of variables.
2) a set of possible values (domain) for each variable.
3) constraints that restrict combinations of allowed values (relations between variables).

A constraint satisfaction problem is solved when there is a complete consistent instantiation of variables, also referred to as a solution. A solution is generally found by inference and search.

In the present example, all the to-be-scheduled content blocks for the various (in-between) scan moments are considered to be variables. Constraints for finding a solution (entertainment content schedule) are:
1) on matching moment type (fixed or dynamic duration) and length (fixed for scans, deduced from Examcard, dynamic for e.g., phases between scans) to content type (loopable y/n, story arch y/n) and length (from curated content database).
2) on matching specifics of patient characteristics to the target group annotations of the content (from curated content database)
3) on matching specifics of 'risky behaviors' by sequence type (from Sequence Database) to 'chance of behavior' of content (from curated content database)
4) on checking specifics of (sum of / scheduled / combined) 'chance of behavior' of content (from curated content database) to 'chance of behavior' derived from the human in-bore behavior model.
5) on checking specifics of (sum of / scheduled / combined) 'chance of behavior' of content (from curated content database) to 'chance of engagement' derived from the human in-bore behavior model.
When all the hard constraints are fulfilled by inference, the values of the remaining open variables can be filled by search.

Preferences (soft constraints), e.g., content X is more likely to cause a pleasant patient experience then content Y, can be explicitly represented by a cost for each variable assignment or implicitly by structuring the search in such a way that preferred options are found first.

In phase 2 the entertainment content schedule is executed in real-time in interaction with the MRI scanner and, if needed, the content blocks are adapted in the timescale seconds to half a minute.

The actual execution of scheduled moments is steered by the content executor 60, which may be a console (computer) of the MRI scanner. When a scan sequence is started by an MR technologist 80 a 'start' signal is received and selected content matching the medical procedure is played according to the entertainment content schedule as provided by the content scheduler 50. In this example, when a sequence is finished, a 'stop' signal is send by the scanner. Three events may occur; the stop signal is received before, at the exact moment, or after the moment the scheduled content is finished.

In any medical procedure it may be necessary to divert from the originally scheduled planned procedural steps. For instance, a step may take longer or shorter than expected, new information may be obtained during the medical procedure that causes new actions to be undertaken, for instance by adding new procedural steps or replacing originally scanned procedural steps or a patient's behavior may cause a medical procedure to run longer or shorter, e.g. because instructions need to be repeated or patient motion is more or less than originally expected, e.g. because a patient is more stressed than expected or if a patient falls asleep during a medical procedure (which is not uncommon in for instance MRI scans).

In such cases the originally planned entertainment content schedule is not relevant anymore as beginning and ending of entertainment blocks is unlikely to be synchronous with the updated procedural steps or the effect of the entertainment blocks on the patient behavior is not suitable, necessary or even detrimental for one or more procedural steps. For instance, if originally an eye gaze behavior was planned at a certain moment in the medical procedure, now the patient, including his eyes, should be completely still or an entertainment block that was supposed to provide relief or even laughter in a noncritical procedural step (e.g. during a procedural step which is a waiting period while a medical professional prepares next procedural step) may now occur during a moment in the procedure in which movement would be very detrimental to the outcome of the medical procedure.

Therefore, it is preferable that the entertainment content provided to the patient is updated during the medical procedure to fit the new or changed procedural step(s).

To achieve this the entertainment content provider 10 receives updated medical procedure data after the original entertainment schedule was created. This may be before or during the execution of the medical procedure. In some embodiments the updated medical procedure data is provided manually by the operator 80. In other embodiments the entertainment content provider 10 comprises logic that (semi-)automatically determines if the medical procedure has changed or predicts if it will change. This may be facilitated by (for instance, camera-based) monitoring of the medical procedure and/or the patient or by analyzing measured data or images obtained during the medical procedure.

The content scheduler 50 compares the updated medical procedure data with the original medical procedural data. The content scheduler 50 preferably checks which procedural steps are added, removed or changed and from what procedural step the original schedule needs to be adapted.

If required because of the update medical procedure, the content scheduler 50 then updates the entertainment content schedule by removing, adding, replacing, reordering or adapting entertainment blocks in a similar manner as with the original selection by matching metadata relating to the risk factor and the expected effect data.

For instance, if a procedural step is longer then it may be necessary to lengthen an existing entertainment block by having it played at a slower speed or by adding a new sub-scene or a new suitable entertainment block. Alternatively the original entertainment block may be replaced if the procedure requires a very different response during the entertainment block (for instance lying still instead of moving a body part or vice versa) or if the length is extended or shortened beyond what it is possible with the original entertainment block (for instance, slowing the playback speed may cause the entertainment content to become unnatural and therefore distracting to the patient).

The content executor 60 receives the updated entertainment content schedule from the content scheduler 50. This may be triggered manually or automatically, for instance by an update signal sent by the content scheduler 50 or because the content executor 60 regularly or continuously checks for updated information from the content scheduler 50.

The content executor adapts currently scheduled entertainment blocks to conform to the updated entertainment schedule, removes any entertainment blocks that are not in the updated entertainment schedule and/or retrieves new entertainment blocks from the curated entertainment content database and plays the entertainment blocks according to the order required by the updated entertainment schedule.

The medical procedure may be updated more than once, therefore the entertainment schedule may need to be updated several times as well.

In the example described previously this may take place as follows. When the 'stop' signal sent by the scanner co-appears with the moment the entertainment block is finished, nothing needs to be done and it is used as the start signal for showing the next scheduled entertainment block, for instance in-between scan content.

When the 'stop' signal is received before the content is finished, the entertainment block's content can either be aborted (and preferably smoothly transition into the next, in-between or next sequence, entertainment content), or continue to run and transition into the next, for instance in-between or next sequence, entertainment content upon finishing. The selection depends on the need for the in-between content based on a patient (in-bore) behavior model. If the in-between content includes an actual function, e.g. show instruction for the next sequence, or actively encouraging swallowing to prevent that from happening during the next sequence, it should not be compromised such that its objective and/or function is lost. This may be determined by checking the relevant step for the two situations with and without the in-between sequence content and the final 'preference' score for the two options.

When the 'stop' signal is not yet received, while the entertainment block has been completely played, a smart real-time adaptation needs to occur to ensure the entertainment content does not simply stop and offers a static (or worse black screen) to the patient, signaling that the sequence is finished although this is not the case. One simple means is, for instance, slowly zooming into the center of the final shot. Alternatively, contents of fixed length come with a, for instance, 30 seconds 'additional' end part that is optional to be shown to accommodate for such variations. Such content is useful for scheduling content with lengths matching procedures to the levels of tens of seconds instead of more fine-grained scheduling. In addition, it is useful for dynamic (e.g. cardiac or respiratory scans) that have varied unknown lengths to start with.

To illustrate this further, in a specific non-limiting example the content schedule is updated in real-time based on changes in the ExamCard or sequence during the medical procedure. The content scheduler 50 may be activated multiple times (on demand, automatically, regularly or continuously) during an examination to update the scheduled content of the entertainment sequence due to changes in the ExamCard or sequences, or due to (manual) operations by the MRI technician 80 or patient behavior that cause an unexpected amount of additional time.

In one case in this example the MRI scan is extended with further sequences, which leads to an addition of content to the content schedule at the correct moment. In another instance of this example a removal of a sequence which only leads to a check by the content scheduler 50 to determine whether the complete schedule still aligns with constraints posed by the human model or if there need to be adaptations (which then may be implemented on-the-fly).

In a further exemplary embodiment of the presently claimed invention interaction between the MRI scanner 90 and content executor 60 goes both ways. In the previous embodiments the MRI scanner 90 (through the MR technologist 80) is fully in charge of when to start a MRI sequence, in this embodiment the content scheduler 60 also has control over when sequences are started.

The main benefit of this embodiment is that when it is determined that, for instance, the chance of detrimental patient behavior is decreased, and the chance of higher patient engagement is increased when certain scheduled entertainment content is fully shown (i.e. not cut short) in the in between sequences time slot, it can force the next sequence to wait although from the MR workflow perspective it could already start.

In a next non-limiting example, the content schedule is updated in real-time based on changes in a (personalized) patient-specific model of in-scanner behavior. This model is updated via information from one or more additional motion or behavior sensors.

Such sensor(s) make measurements of the patient, either directly via additional hardware (e.g. an optical or infra-red camera, eye-tracking, heart-rate ECG, a respiratory belt, PPU, pressure sensors, etc.) or indirectly via measurement made by the MRI scanner (e.g. realignment of images, reconstruction of K-space, etc.). These measurements quantify patient-specific in-bore behavior. This may include voluntary behavior such as motion of the head, limbs, chest, legs or facial expressions, as well as in-voluntary in-bore behavior, such as blinking, coughing and swallowing, an/or patient-specific physiology, including heart rate, breathing patterns and galvanic skin conduction.

The sensory-derived measurements of in-bore behaviors are incorporated by a patient behavior module 40. This module 40 can use a patient-specific model that can detect, or even predict, relevant behavioral changes of the patient in real-time and in a relatively straightforward manner. This is done by implementing a limited number of crude rules to specify behavior. For example, the patient behavior module 40 can estimate an amount of patient motion during scan acquisition using alignment of consecutive MRI images, using a video camera or pressure sensors. If a patient shows a relatively large amount of head motion, this data may be transmitted to the content scheduler 50, which could prioritize content that helps focus the eyes and reduce head motion. Or, if the breathing-rate keeps increasing during the initial part of the scans, the module 40 could provide the content scheduler 50 with this information, which then could prioritize content that helps relax and reduce the breathing rate.

The patient behavior module 40 also may receive prior behavior patient data from the operator or patient before or during the medical procedure. This prior or in-procedure behavior information data may comprises normative in-scanner behavior data and places measured behavior in context of a reference. The prior information helps to correctly interpret in-scanner behavior and detect what is normal or abnormal. This comparison can be done relative to static reference or stratified to age, weight, BMI, gender or a patient group. For example, head motion is best quantified relative to age, as young children tend to move more. This stratification relative to age, is then also explicitly passed to the content scheduler to help select content from the annotated content database.

In a further non-limiting example building on the previous, a more sophisticated, machine learning-based model of patient specific in-bore behavior could also be deployed by the patient behavior module 40. A machine-learning based model would need to be trained to directly map sensory measurement to content, and thereby include the prior information. When deployed after training, this model would directly suggest content based on sensory information.

In a further non-limiting example, instead of a global estimation of the duration of the intra-scan-intervals based on common knowledge available within the content scheduler 50, this knowledge may come from a specific separate component. This component may, besides this global knowledge, also embed more specific, personalized knowledge (from expert knowledge, library data or learned from such data). For instance, this component is aware that technologist X takes on average x minutes to plan an examination type a, while tech Y takes y minutes on average.

In some procedures it may be necessary to interrupt the entertainment sequence to provide communicate with the patient, for instance to provide additional instructions or to insert a functional content block that is not available within the current storyline, for instance a breathing coach. In those cases it is preferable to provide a smooth transition out of the entertainment content into the functional content or out of the content completely. This may be done by fading the images and sound or to provide a, preferably subtle, notification that the entertainment will be interrupted. In some cases there may be transitional entertainment blocks specific for this purpose, for instance a character that speaks directly to the patient to inform about the temporary interruption.

The components of the adaptive entertainment scheduler may be integrated into one processor or other computing device or may be spread over various processors or computing devices connected wired or wirelessly. For instance, the medical procedure database 20 and the curated content database 30 may be on a remote data storage device, for instance a separate on-site or off-site data station or it may be in a cloud-based data storage.

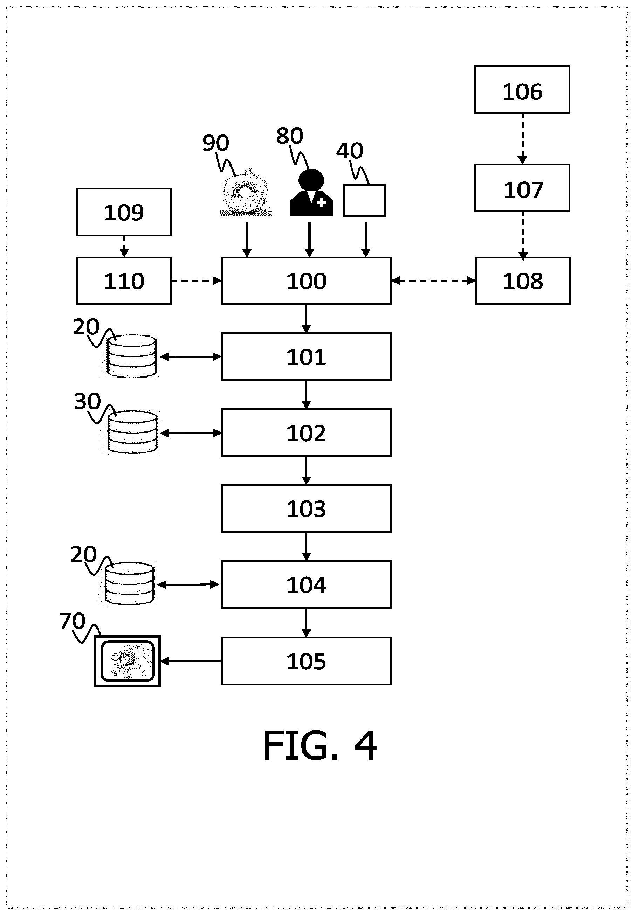

Fig. 4 depicts a flowchart of an embodiment of a method for providing an entertainment sequence to a patient during a medical procedure according to the presently claimed invention. First, medical procedure data of the medical procedure is received 100 from medical equipment 90, an operator or physician 80 and/or a patient behavior module 40.

Next, the procedural step database 20 is consulted 101 and the procedural step options are selected.

Next, the curated content database 30 is consulted 102 to select entertainment blocks by matching the risk factor of the selected procedural options with a most suitable predetermined expected effect data of an entertainment block.

Next, the entertainment content schedule is created 103 by listing the selected entertainment blocks based on the order of the procedural steps in the succession of procedural steps.

Next, the selected entertainment blocks in the content schedule are retrieved 104 from the curated content database 20.

Next, entertainment content is provided 105 to the patient on a content delivery device 70 (e.g. a display, goggles, headphones and the like) during the medical procedure by presenting the retrieved entertainment blocks according to the entertainment content schedule.

In case the medical procedure is updated 106, for instance by changing, adding or removing a procedural step, then updated medical procedure data is received 107 and compared 108 with the medical procedure data. In case any of the procedural steps has changed, has been removed and/or added, the previously mentioned process is repeated to adjust the enterainment sequence using adapted or new entertainment blocks or removal of entertainment blocks.

Preferably, patient behavior data relating to the medical procedure is taken into account 109 prior to the procedure, this may be manually input by the patient, operator/physician 80 or the patient behavior module 40. This patient behavior data is then used 110 to decide which entertainment blocks are selected.

While the invention has been illustrated and described in detail in the drawings and foregoing description, such illustration and description are to be considered illustrative or exemplary and not restrictive; the invention is defined by the appended claims.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. Entertainment content provider for providing an entertainment sequence to a patient during a medical procedure, wherein the medical procedure comprises a succession of procedural steps, comprising:
- a procedural step database comprising a catalogue of procedural steps associated with the medical procedure and wherein each procedural step in the catalogue includes procedural step metadata including:
- a procedural step identifier;
- a procedural step length; and
- at least one risk factor that is based on a previously determined detrimental effect of patient behavior on the outcome of the medical procedure;
- a curated content database, comprising a catalogue of entertainment blocks, wherein each entertainment block includes:
- entertainment content; and
- metadata including:
- an entertainment block length; and
- predetermined expected effect data on one or more procedural steps from the procedural step database;
- a content scheduler, configured to
- receive medical procedure data of the medical procedure, comprising data relating to the procedural steps in the succession of procedural steps, including an order of the procedural steps in the succession of procedural steps;
- consult the procedural step database and select procedural step options corresponding with the successive procedural steps of the medical procedure;
- consult the curated content database and select entertainment blocks by matching the risk factor of the selected procedural options with a most suitable predetermined expected effect data of an entertainment block;
- create an entertainment content schedule by listing the selected entertainment blocks based on the order of the procedural steps in the succession of procedural steps; and
- a content executor, configured to:
- receive the entertainment content schedule from the content scheduler;
- retrieve the selected entertainment blocks in the content schedule from the curated content database;
- provide entertainment content to the patient during the medical procedure by presenting the retrieved entertainment blocks according to the entertainment content schedule.

2. Entertainment content provider according to claim 1, wherein the content scheduler is further configured to also select entertainment blocks by matching or adapting an entertainment block length to a procedural step length.

3. Entertainment content provider according to claim 1 or 2, wherein the entertainment blocks further comprise metadata relating to storyline priority and wherein the content scheduler is further configured to also select entertainment blocks based on the storyline priority metadata.

4. Entertainment content provider according to any of the previous claims, wherein the entertainment block length is fixed, adaptable or dynamic.

5. Entertainment content provider according to any of the previous claims, wherein the entertainment block further includes patient target group information indicating for which patient target group the entertainment content is most suitable, for instance a patient target group based on age or age group, gender, cognitive ability, eye sight, hearing, literacy, diagnosis, special interests and the like and the content scheduler is further configured to also select entertainment blocks based on the patient target group information.

6. Entertainment content provider according to any of the previous claims, wherein the content scheduler is further configured to:
- receive updated medical procedure data before or during the medical procedure, comprising updated data relating to the procedural steps in the succession of procedural steps;
- compare the updated medical procedure data with the medical procedure data; and
- in case any of the procedural steps has changed, has been removed and/or added:
- consult the procedural step database and select the procedural step options relating to the successive procedural steps of the updated medical procedure;
- consult the curated content database and select entertainment blocks based on the selected procedural options;
- update the entertainment content schedule by listing the selected entertainment blocks based on the order of the procedural steps in the succession of procedural steps of the updated medical procedure.

7. Entertainment content provider according to any of the previous claims, further comprising:
- a patient behavior module, comprising:
- a patient behavior data receiver configured to receive patient behavior data relating to the medical procedure, preferably the patient behavior data is previously obtained patient behavior data, library patient data, modeled patient behavior and/or real-time acquired patient behavior data.;
- a patient behavior data provider for providing the patient behavior data to the content scheduler; and wherein the content scheduler is further configured to receive the patient behavior data from the patient behavior module and to consult the curated content database and select entertainment blocks also based on the patient behavior data.

8. Entertainment content provider according to claim 7 or 8, wherein a neural network is configured to analyze the previously obtained patient behavior data, library patient data, modeled patient behavior data and/or real-time acquired patient behavior data to generate expected patient behavior data forming or replacing the patient behavior data.

9. Entertainment content provider according to any of the previous claims, wherein the content executor is further configured to
- receive updated medical procedure data;
- adapt the entertainment schedule by adapting, removing or adding at least one entertainment content blocks; and
- provide the adapted entertainment content.

10. Entertainment content provider according to any of the claims claim 1 to 10, wherein the medical procedure is a magnetic resonance imaging (MRI) procedure and the succession of procedural steps includes at least one MRI sequence.

11. Method for providing an entertainment sequence to a patient during a medical procedure, wherein the medical procedure comprises a succession of procedural steps, the method comprising:
- receiving (100) medical procedure data of the medical procedure, comprising data relating to the procedural steps in the succession of procedural steps, including an order of the procedural steps in the succession of procedural steps;
- consulting (101) a procedural step database and select the procedural step options corresponding with the successive procedural steps of the medical procedure, wherein the procedural step database comprises:
- a catalogue of procedural steps associated with the medical procedure and wherein each procedural step in the catalogue includes procedural step metadata including:
- a procedural step identifier;
- a procedural step length; and
- at least one risk factor that is based on a previously determined detrimental effect of patient behavior on the outcome of the medical procedure
- consulting (102) a curated content database to select entertainment blocks by matching the risk factor of the selected procedural options with a most suitable predetermined expected effect data of an entertainment block, wherein the curated content database comprises:
- a catalogue of entertainment blocks, wherein each entertainment block includes:
- entertainment content; and
- metadata including:
- an entertainment block length; and
- predetermined expected effect data on one or more procedural steps from the procedural step database;
- creating (103) an entertainment content schedule by listing the selected entertainment blocks based on the order of the procedural steps in the succession of procedural steps;
- retrieving (104) the selected entertainment blocks in the content schedule from the curated content database;
- providing (105) entertainment content to the patient during the medical procedure by presenting the retrieved entertainment blocks according to the entertainment content schedule.

12. Method according to claim 11, further comprising the steps of:
- receiving (107) updated medical procedure data before or during the medical procedure, comprising updated data relating to the procedural steps in the succession of procedural steps;
- comparing (108) the updated medical procedure data with the medical procedure data; and
- in case any of the procedural steps has changed, has been removed and/or added:
- consulting (101) the procedural step database and select the procedural step options relating to the successive procedural steps of the updated medical procedure;
- consulting (102) the curated content database and select entertainment blocks based on the selected procedural options;
- updating (103) the entertainment content schedule by listing the selected entertainment blocks based on the order of the procedural steps in the succession of procedural steps of the updated medical procedure.

13. Method according to claims 11 or 12, further comprising the steps of:
- receiving (109) patient behavior data relating to the medical procedure;
- additionally using (110) the patient behavior data to select entertainment blocks.

14. Method for providing adaptive entertainment content according to any of the claims 11 tot 13, wherein the medical procedure is a magnetic resonance imaging (MRI) procedure and the succession of procedural steps includes at least one MRI sequence.

15. Computer program product configured to execute the steps of a method according to any of the claims 11 to 13.

## Patentansprüche

1. Anbieter von Unterhaltungsinhalten zum Bereitstellen einer Unterhaltungssequenz an einen Patienten während einer medizinischen Prozedur, wobei die medizinische Prozedur eine Abfolge von Prozedurschritten umfasst, umfassend:
- eine Prozedurschrittdatenbank, die einen Katalog von Prozedurschritten umfasst, die der medizinischen Prozedur zugeordnet sind, und wobei jeder Prozedurschritt in dem Katalog Prozedurschritt-Metadaten beinhaltet, beinhaltend:
- eine Prozedurschrittkennung;
- eine Prozedurschrittlänge; und
- mindestens einen Risikofaktor, der auf einer zuvor festgestellten nachteiligen Wirkung von Patientenverhalten auf das Ergebnis der medizinischen Prozedur beruht;
- eine kuratierte Inhaltsdatenbank, die einen Katalog von Unterhaltungsblöcken umfasst, wobei jeder Unterhaltungsblock Folgendes beinhaltet:
- Unterhaltungsinhalte; und
- Metadaten, beinhaltend:
- eine Unterhaltungsblocklänge; und
- vorbestimmte Daten zu der erwarteten Wirkung auf einen oder mehrere Prozedurschritte aus der Prozedurschrittdatenbank;
- einen Inhaltsplaner, der dazu eingerichtet ist,
- medizinische Prozedurdaten der medizinischen Prozedur zu empfangen, die Daten in Bezug auf die Prozedurschritte in der Abfolge von Prozedurschritten umfassen, beinhaltend eine Reihenfolge der Prozedurschritte in der Abfolge von Prozedurschritten;
- die Prozedurschrittdatenbank einzusehen und Prozedurschrittoptionen auszuwählen, die den aufeinanderfolgenden Prozedurschritten der medizinischen Prozedur entsprechen;
- die kuratierte Inhaltsdatenbank einzusehen und Unterhaltungsblöcke auszuwählen, indem der Risikofaktor der ausgewählten Prozeduroptionen mit am besten geeigneten vorbestimmten Daten zu der erwarteten Wirkung eines Unterhaltungsblocks abgestimmt wird;
- eine Unterhaltungsinhaltsplanung anzulegen, indem die ausgewählten Unterhaltungsblöcke basierend auf der Reihenfolge der Prozedurschritte in der Abfolge von Prozedurschritten aufgelistet werden; und
- einen Inhaltsausführer, der dazu eingerichtet ist:
- die Unterhaltungsinhaltsplanung von dem Inhaltsplaner zu erhalten;
- die ausgewählten Unterhaltungsblöcke in die Inhaltsplanung aus der kuratierten Inhaltsdatenbank abzurufen;
- Unterhaltungsinhalte an den Patienten während der medizinischen Prozedur durch Präsentieren der abgerufenen Unterhaltungsblöcke gemäß der Unterhaltungsinhaltsplanung bereitzustellen.

2. Anbieter von Unterhaltungsinhalten nach Anspruch 1, wobei der Inhaltsplaner weiter dazu eingerichtet ist, auch Unterhaltungsblöcke auszuwählen, indem er eine Unterhaltungsblocklänge auf eine Prozedurschrittlänge abstimmt oder daran anpasst.

3. Anbieter von Unterhaltungsinhalten nach Anspruch 1 oder 2, wobei die Unterhaltungsblöcke weiter Metadaten in Bezug auf die Handlungspriorität umfassen, und wobei der Inhaltsplaner weiter dazu eingerichtet ist, auch Unterhaltungsblöcke basierend auf den Handlungspriorität-Metadaten auszuwählen.

4. Anbieter von Unterhaltungsinhalten nach einem der vorstehenden Ansprüche, wobei die Unterhaltungsblocklänge fest, anpassbar oder dynamisch ist.

5. Anbieter von Unterhaltungsinhalten nach einem der vorstehenden Ansprüche, wobei der Unterhaltungsblock weiter Patientenzielgruppen-Informationen beinhaltet, die angeben, für welche Patientenzielgruppe der Unterhaltungsinhalt am besten geeignet ist, beispielsweise eine Patientenzielgruppe basierend auf Alter oder Altersgruppe, Geschlecht, kognitiven Fähigkeiten, Sehvermögen, Gehör, Lese- und Schreibfähigkeit, Diagnose, besonderen Interessen und dergleichen, und wobei der Inhaltsplaner weiter dazu eingerichtet ist, auch Unterhaltungsblöcke basierend auf den Patientenzielgruppen-Informationen auszuwählen.

6. Anbieter von Unterhaltungsinhalten nach einem der vorstehenden Ansprüche, wobei der Inhaltsplaner weiter dazu eingerichtet ist:
- vor oder während der medizinischen Prozedur aktualisierte Daten zu der medizinischen Prozedur zu erhalten, die aktualisierte Daten in Bezug auf die Prozedurschritte in der Abfolge von Prozedurschritten umfassen;
- die aktualisierten Daten zu der medizinischen Prozedur mit den Daten zu der medizinischen Prozedur zu vergleichen; und
- falls sich einer der Prozedurschritte geändert hat, entfernt und/oder hinzugefügt wurde:
- die Datenbank mit den Prozedurschritten einzusehen und die Prozedurschrittoptionen in Bezug auf die aufeinanderfolgenden Prozedurschritte der aktualisierten medizinischen Prozedur auszuwählen;
- die kuratierte Inhaltsdatenbank einzusehen und Unterhaltungsblöcke basierend auf den ausgewählten Prozeduroptionen auszuwählen;
- die Unterhaltungsinhaltplanung durch Auflisten der ausgewählten Unterhaltungsblöcke basierend auf der Reihenfolge der Prozedurschritte in der Abfolge von Prozedurschritten der aktualisierten medizinischen Prozedur zu aktualisieren.

7. Anbieter von Unterhaltungsinhalten nach einem der vorstehenden Ansprüche, weiter umfassend:
- ein Patientenverhaltensmodul, das Folgendes umfasst:
- einen Patientenverhaltensdatenempfänger, der dazu eingerichtet ist, Patientenverhaltensdaten in Bezug auf die medizinische Prozedur zu empfangen, wobei die Patientenverhaltensdaten bevorzugt zuvor erhaltene Patientenverhaltensdaten, Bibliothekpatientendaten, modelliertes Patientenverhalten und/oder in Echtzeit erhobene Daten zum Patientenverhalten sind;
- einen Patientenverhaltensdatenanbieter zum Bereitstellen der Patientenverhaltensdaten an den Inhaltsplaner; und wobei der Inhaltsplaner weiter dazu eingerichtet ist, die Patientenverhaltensdaten von dem Patientenverhaltensmodul zu empfangen und die kuratierte Inhaltsdatenbank einzusehen und Unterhaltungsblöcke ebenfalls basierend auf den Patientenverhaltensdaten auszuwählen.

8. Anbieter von Unterhaltungsinhalten nach Anspruch 7 oder 8, wobei ein neuronales Netzwerk dazu eingerichtet ist, die zuvor erhaltenen Patientenverhaltensdaten, Bibliothekpatientendaten, modellierten Patientenverhaltensdaten und/oder in Echtzeit erhobenen Patientenverhaltensdaten zu analysieren, um erwartete Patientenverhaltensdaten, die die Patientenverhaltensdaten bilden oder ersetzen, zu erzeugen.

9. Anbieter von Unterhaltungsinhalten nach einem der vorstehenden Ansprüche, wobei der Inhaltsausführer weiter dazu eingerichtet ist,
- aktuelle Daten zur medizinischen Prozedur zu empfangen;
- die Unterhaltungsinhaltsplanung durch Anpassen, Entfernen oder Hinzufügen mindestens eines Unterhaltungsinhaltsblocks anzupassen; und
- angepasste Unterhaltungsinhalte bereitzustellen.

10. Anbieter von Unterhaltungsinhalten nach einem der Ansprüche 1 bis 10, wobei die medizinische Prozedur eine Magnetresonanztomographie- (MRT) Prozedur ist und die Abfolge der Prozedurschritte mindestens eine MRT-Sequenz beinhaltet.

11. Verfahren zum Bereitstellen einer Unterhaltungssequenz an einen Patienten während einer medizinischen Prozedur, wobei die medizinische Prozedur eine Abfolge von Prozedurschritten umfasst, wobei das Verfahren Folgendes umfasst:
- Empfangen (100) medizinischer Prozedurdaten der medizinischen Prozedur, die Daten in Bezug auf die Prozedurschritte in der Abfolge von Prozedurschritten umfassen, beinhaltend eine Reihenfolge der Prozedurschritte in der Abfolge von Prozedurschritten;
- Einsehen (101) einer Prozedurschrittdatenbank und Auswählen der Prozedurschrittoptionen, die den aufeinanderfolgenden Prozedurschritten der medizinischen Prozedur entsprechen, wobei die Prozedurschrittdatenbank Folgendes umfasst:
- einen Katalog von Prozedurschritten, die der medizinischen Prozedur zugeordnet sind, und wobei jeder Prozedurschritt in dem Katalog Prozedurschritt-Metadaten beinhaltet, beinhaltend:
- eine Prozedurschrittkennung;
- eine Prozedurschrittlänge; und
- mindestens einen Risikofaktor, der auf einer zuvor festgestellten nachteiligen Wirkung von Patientenverhalten auf das Ergebnis der medizinischen Prozedur beruht;
- Einsehen (102) einer kuratierten Inhaltsdatenbank zum Auswählen von Unterhaltungsblöcken durch Abstimmen des Risikofaktors der ausgewählten Prozeduroptionen mit den am besten geeigneten vorbestimmten erwarteten Wirkungsdaten eines Unterhaltungsblocks, wobei die kuratierte Inhaltsdatenbank Folgendes umfasst:
- einen Katalog von Unterhaltungsblöcken, wobei jeder Unterhaltungsblock Folgendes beinhaltet:
- Unterhaltungsinhalte; und
- Metadaten, beinhaltend:
- eine Unterhaltungsblocklänge; und
- vorbestimmte Daten zu der erwarteten Wirkung auf einen oder mehrere Prozedurschritte aus der Prozedurschrittdatenbank;
- Anlegen (103) einer Unterhaltungsinhaltsplanung durch Auflisten der ausgewählten Unterhaltungsblöcke basierend auf der Reihenfolge der Prozedurschritte in der Abfolge von Prozedurschritten;
- Abrufen (104) der ausgewählten Unterhaltungsblöcke in der Inhaltsplanung aus der kuratierten Inhaltsdatenbank;
- Bereitstellen (105) von Unterhaltungsinhalten an den Patienten während der medizinischen Prozedur durch Präsentieren der abgerufenen Unterhaltungsblöcke gemäß der Unterhaltungsinhaltsplanung.

12. Verfahren nach Anspruch 11, das weiter die folgenden Schritte umfasst:
- Empfangen (107) aktualisierter medizinischer Prozedurdaten vor oder während der medizinischen Prozedur, die aktualisierte Daten in Bezug auf die Prozedurschritte in der Abfolge von Prozedurschritten umfassen;
- Vergleichen (108) der aktualisierten medizinischen Prozedurdaten mit den medizinischen Prozedurdaten; und
- falls sich einer der Prozedurschritte geändert hat, entfernt und/oder hinzugefügt wurde:
- Einsehen (101) der Prozedurschrittdatenbank und Auswählen der Prozedurschrittoptionen in Bezug auf die aufeinanderfolgenden Prozedurschritte der aktualisierten medizinischen Prozedur;
- Einsehen (102) der kuratierten Inhaltsdatenbank und Auswählen von Unterhaltungsblöcken basierend auf den ausgewählten Prozeduroptionen;
- Aktualisieren (103) der Unterhaltungsinhaltsplanung durch Auflisten der ausgewählten Unterhaltungsblöcke basierend auf der Reihenfolge der Prozedurschritte in der Abfolge von Prozedurschritten der aktualisierten medizinischen Prozedur.

13. Verfahren nach Anspruch 11 oder 12, das weiter die folgenden Schritte umfasst:
- Empfangen (109) von Patientenverhaltensdaten in Bezug auf die medizinische Prozedur;
- zusätzlich Verwenden (110) der Patientenverhaltensdaten zum Auswählen von Unterhaltungsblöcken.

14. Verfahren zum Bereitstellen adaptiver Unterhaltungsinhalte nach einem der Ansprüche 11 bis 13, wobei die medizinische Prozedur eine Magnetresonanztomographie- (MRT) Prozedur ist und die Abfolge der Prozedurschritte mindestens eine MRT-Sequenz beinhaltet.

15. Computerprogrammprodukt, das dazu eingerichtet ist, die Schritte eines Verfahrens nach einem der Ansprüche 11 bis 13 auszuführen.

## Revendications

1. Fournisseur de contenu de divertissement destiné à fournir une séquence de divertissement à un patient durant une procédure médicale, dans lequel la procédure médicale comprend une succession d'étapes procédurales, comprenant :
- une base de données d'étapes procédurales comprenant un catalogue d'étapes procédurales associées à la procédure médicale et dans laquelle chaque étape procédurale du catalogue inclut des métadonnées d'étape procédurale comprenant :
- un identifiant d'étape procédurale ;
- une longueur d'étape procédurale ; et
- au moins un facteur de risque qui est fondé sur un effet néfaste préalablement déterminé d'un comportement de patient sur le résultat de la procédure médicale ;
- une base de données de contenu organisé, comprenant un catalogue de blocs de divertissement, dans laquelle chaque bloc de divertissement inclut :
- un contenu de divertissement ; et
- des métadonnées incluant :
- une longueur de bloc de divertissement ; et
- des données d'effet escompté prédéterminées sur une ou plusieurs étapes procédurales issues de la base de données d'étapes procédurales ;
- un programmateur de contenu, configuré pour
- recevoir des données de procédure médicale de la procédure médicale, comprenant des données relatives aux étapes procédurales de la succession d'étapes procédurales, incluant un ordre des étapes procédurales dans la succession d'étapes procédurales ;
- consulter la base de données d'étapes procédurales et sélectionner des options d'étapes procédurales correspondant aux étapes procédurales successives de la procédure médicale ;
- consulter la base de données de contenu organisé et sélectionner des blocs de divertissement en faisant correspondre le facteur de risque des options procédurales sélectionnées avec les données d'effet escompté prédéterminées les plus appropriées d'un bloc de divertissement ;
- créer un programme de contenu de divertissement en listant les blocs de divertissement sélectionnés en fonction de l'ordre des étapes procédurales de la succession d'étapes procédurales ; et
- un exécuteur de contenu, configuré pour :
- recevoir le programme de contenu de divertissement du programmateur de contenu ;
- récupérer les blocs de divertissement sélectionnés dans le programme de contenu à partir de la base de données de contenu organisé ;
- fournir un contenu de divertissement au patient durant la procédure médicale en présentant les blocs de divertissement récupérés en fonction du programme de contenu de divertissement.

2. Fournisseur de contenu de divertissement selon la revendication 1, dans lequel le programmateur de contenu est en outre configuré pour sélectionner également des blocs de divertissement en faisant correspondre ou en adaptant une longueur de bloc de divertissement avec ou à une longueur d'étape procédurale.

3. Fournisseur de contenu de divertissement selon la revendication 1 ou 2, dans lequel les blocs de divertissement comprennent en outre des métadonnées relatives à la priorité du scénario et dans lequel le programmateur de contenu est en outre configuré pour sélectionner également des blocs de divertissement en fonction des métadonnées de priorité du scénario.

4. Fournisseur de contenu de divertissement selon l'une quelconque des revendications précédentes, dans lequel la longueur de bloc de divertissement est fixe, adaptable, ou dynamique.

5. Fournisseur de contenu de divertissement selon l'une quelconque des revendications précédentes, dans lequel le bloc de divertissement inclut en outre des informations de groupe cible de patients indiquant le groupe cible de patients pour lequel le contenu de divertissement est le plus approprié, par exemple un groupe cible de patients fondé sur l'âge ou un groupe d'âge, le sexe, la capacité cognitive, la vue, l'audition, le degré d'alphabétisation, le diagnostic, les intérêts spéciaux et analogues et le programmateur de contenu est en outre configuré pour sélectionner également des blocs de divertissement sur la base des informations de groupe cible de patients.

6. Fournisseur de contenu de divertissement selon l'une quelconque des revendications précédentes, dans lequel le programmateur de contenu est en outre configuré pour :
- recevoir des données de procédure médicale mises à jour, avant ou durant la procédure médicale, comprenant des données mises à jour relatives aux étapes procédurales de la succession d'étapes procédurales ;
- comparer les données de procédure médicale mises à jour aux données de procédure médicale ; et
- au cas où une quelconque des étapes procédurales aurait changé, aurait été supprimée et/ou ajoutée :
- consulter la base de données d'étapes procédurales et sélectionner les options d'étapes procédurales relatives aux étapes procédurales successives de la procédure médicale mis à jour ;
- consulter la base de données de contenu organisé et sélectionner les blocs de divertissement en fonction des options procédurales sélectionnées ;
- mettre à jour le programme de contenu de divertissement en listant les blocs de divertissement sélectionnés en fonction de l'ordre des étapes procédurales de la succession d'étapes procédurales de la procédure médicale mise à jour.

7. Fournisseur de contenu de divertissement selon l'une quelconque des revendications précédentes, comprenant en outre :
- un module de comportement de patient, comprenant :
- un récepteur de données de comportement de patient configuré pour recevoir des données de comportement de patient relatives à la procédure médicale, de préférence les données de comportement de patient sont des données de comportement de patient préalablement obtenues, des données de patient de bibliothèque, des données de comportement de patient modélisées et/ou de comportement de patient acquises en temps réel. ;
- un fournisseur de données de comportement de patient pour fournir les données de comportement de patient au programmateur de contenu ; et dans lequel le programmateur de contenu est en outre configuré pour recevoir les données de comportement de patient en provenance du module de comportement de patient et pour consulter la base de données de contenu organisé et sélectionner des blocs de divertissement également en fonction des données de comportement de patient.

8. Fournisseur de contenu de divertissement selon la revendication 7 ou 8, dans lequel un réseau neuronal est configuré pour analyser les données de comportement de patient préalablement obtenues, les données de patient de bibliothèque, les données de comportement de patient modélisées et/ou données de comportement de patient acquises en temps réel pour générer des données de comportement de patient escompté formant ou remplaçant les données de comportement de patient.

9. Fournisseur de contenu de divertissement selon l'une quelconque des revendications précédentes, dans lequel l'exécuteur de contenu est en outre configuré pour
- recevoir des données de procédure médicale mises à jour ;
- adapter la programmation de divertissement en adaptant, supprimant, ou ajoutant au moins un bloc de contenu de divertissement ; et
- fournir le contenu de divertissement adapté.

10. Fournisseur de contenu de divertissement selon l'une quelconque des revendications 1 à 10, dans lequel la procédure médicale est une procédure d'imagerie par résonance magnétique (IRM) et la succession d'étapes procédurales inclut au moins une séquence IRM.

11. Procédé pour fournir une séquence de divertissement à un patient durant une procédure médicale, dans lequel la procédure médicale comprend une succession d'étapes procédurales, le procédé comprenant :
- la réception (100) de données de procédure médicale de la procédure médicale, comprenant des données relatives aux étapes procédurales de la succession d'étapes procédurales, incluant un ordre des étapes procédurales dans la succession d'étapes procédurales ;
- la consultation (101) d'une base de données d'étapes procédurales et la sélection des options d'étapes procédurales correspondant aux étapes procédurales successives de la procédure médicale, dans lequel la base de données d'étapes procédurales comprend ;
- un catalogue d'étapes procédurales associées à la procédure médicale et dans lequel chaque étape procédurale du catalogue inclut des métadonnées d'étape procédurale comprenant :
- un identifiant d'étape procédurale ;
- une longueur d'étape procédurale ; et
- au moins un facteur de risque qui est fondé sur un effet néfaste préalablement déterminé d'un comportement de patient sur le résultat de la procédure médicale
- la consultation (102) d'une base de données de contenu organisé pour sélectionner des blocs de divertissement en faisant correspondre le facteur de risque des options procédurales sélectionnées avec des données d'effet escompté prédéterminées les plus appropriées d'un bloc de divertissement, dans lequel la base de données de contenu organisé comprend :
- un catalogue de blocs de divertissement, dans lequel chaque bloc de divertissement comprend :
- un contenu de divertissement ; et
- des métadonnées incluant :
- une longueur de bloc de divertissement ; et
- des données d'effet escompté prédéterminées sur une ou plusieurs étapes procédurales issues de la base de données d'étapes procédurales ;
- la création (103) d'un programme de contenu de divertissement en listant les blocs de divertissement sélectionnés en fonction de l'ordre des étapes procédurales de la succession d'étapes procédurales ;
- la récupération (104) des blocs de divertissement sélectionnés dans le programme de contenu à partir de la base de données de contenu organisé ;
- la fourniture (105) d'un contenu de divertissement au patient durant la procédure médicale en présentant les blocs de divertissement récupérés conformément au programme de contenu de divertissement.

12. Procédé selon la revendication 11, comprenant en outre les étapes consistant à :
- recevoir (107) des données de procédure médicale mises à jour, avant ou durant la procédure médicale, comprenant des données mises à jour relatives aux étapes procédurales de la succession d'étapes procédurales ;
- comparer (108) les données de procédure médicale mises à jour aux données de procédure médicale ; et
- au cas où une quelconque des étapes procédurales aurait changé, aurait été supprimée et/ou ajoutée :
- consulter (101) la base de données d'étapes procédurales et sélectionner les options d'étapes procédurales relatives aux étapes procédurales successives de la procédure médicale mis à jour ;
- consulter (102) la base de données de contenu organisé et sélectionner des blocs de divertissement en fonction des options procédurales sélectionnées ;
- mettre à jour (103) le programme de contenu de divertissement en listant les blocs de divertissement sélectionnés en fonction de l'ordre des étapes procédurales de la succession d'étapes procédurales de la procédure médicale mise à jour.

13. Procédé selon les revendications 11 ou 12, comprenant en outre les étapes consistant à :
- recevoir (109) des données de comportement de patient relatives à la procédure médicale ;
- utiliser (110) en plus les données de comportement de patient pour sélectionner des blocs de divertissement.

14. Procédé pour fournir un contenu de divertissement adaptatif selon l'une quelconque des revendications 11 à 13, dans lequel la procédure médicale est une procédure d'imagerie par résonance magnétique (IRM) et la succession d'étapes procédurales inclut au moins une séquence IRM.

15. Produit programme informatique configuré pour exécuter les étapes d'un procédé selon l'une quelconque des revendications 11 à 13.
